# EUROPEAN PATENT SPECIFICATION

(11) **EP 3 614 147 B1**
(45) Date of publication and mention of the grant of the patent: **20.07.2022**
(21) Application number: 18787670.1
(22) Date of filing: 20.04.2018
(51) Int. Cl.: G01N 33/543, C08K 3/08, C08K 3/22, C08K 3/30, C08L 9/00, C08L 25/06, C12Q 1/68, G01N 33/532, G01N 33/533

(54) **METHOD OF DETECTING SPECIMEN SUBSTANCE USING MULTIPHASE POLYMER FINE PARTICLES**
VERFAHREN ZUM NACHWEIS EINER PROBENSUBSTANZ UNTER VERWENDUNG VON FEINPARTIKELN AUS MEHRPHASIGEN POLYMEREN
PROCÉDÉ DE DÉTECTION D'UNE SUBSTANCE SPÉCIMEN À L'AIDE DE FINES PARTICULES DE POLYMÈRE À PLUSIEURS PHASES

(30) Priority: 21.04.2017 JP 2017084812
(43) Date of publication of application: 26.02.2020
(73) Proprietor: Haplopharma Inc., Sendai-shi, Miyagi 980-0845 (JP); Tohoku University, Sendai-shi, Miyagi 980-8577 (JP)
(72) Inventor: SATO Fumitoshi, Sendai-shi Miyagi 980-8577 (JP); YABU Hiroshi, Sendai-shi Miyagi 980-8577 (JP); INOUE Kumi, Sendai-shi Miyagi 980-8577 (JP); SATO Satsuki, Sendai-shi Miyagi 980-8577 (JP); SHIKU Hitoshi, Sendai-shi Miyagi 980-8577 (JP); NEMOTO Yasuhisa, Sendai-shi Miyagi 980-8577 (JP); MAEDA Ikuma, Tokyo 103-0027 (JP)
(74) Representative: Mewburn Ellis LLP
(86) International application number: PCT/JP2018/016261
(87) International publication number: WO 2018/194151

(56) References cited:
- WO-A1-2007/088957
- WO-A1-2016/134115
- JP-A- H09 504 308
- JP-A- 2006 170 853
- JP-A- 2010 185 023
- KYUNG-HO ROH ET AL: "Biphasic Janus particles with nanoscale anisotropy", NATURE MATERIALS, vol. 4, no. 10, 25 September 2005 (2005-09-25), pages 759-763, XP055010513, ISSN: 1476-1122, DOI: 10.1038/nmat1486
- LAN JINGWEN ET AL: "Microfluidic generation of magnetic-fluorescent Janus microparticles for biomolecular detection", TALANTA, vol. 151, 13 January 2016 (2016-01-13), pages 126-131, XP029450844, ISSN: 0039-9140, DOI: 10.1016/J.TALANTA.2016.01.024
- SUCI, PA et al.: "A streptavidin-protein cage Janus particle for polarized targeting and modular functionalizaion", J. AM. CHEM. SOC., vol. 131, no. 26, 2009, pages 9164-9165, XP009137173,
- YABU, H. et al.: "Double-phase-functionalized magnetic Janus polymer microparticles containing Ti02 and Fe203 nanoparticles encapsulated in mussel-inspired amphiphilic polymers", ACS Appl. Mater. Interfaces, vol. 6, 29 September 2014 (2014-09-29), pages 18122-18128, XP055558627,
- FAKHRUDDIN, ST et al.: "2P014 Development of a Janus Particles-utilizing Aldosterone Immunosensor", Proceedings of 2017 Tohoku Conference of Chemical Society of Japan; September 16-17, 2017, vol. 139, 16 September 2017 (2017-09-16), page 2P014, XP009517487,

## Description

### Technical Field

The present invention relates to novel multiphase polymer fine particles, and a method of detecting a specimen substance using the fine particles. More particularly, the present invention provides a technology of detecting and analyzing the specimen substance having a biological origin or the like with high sensitivity, by using a nano-sized, multiphase polymer fine particles.

### Background Art

An anisotropic particle having a plurality of, in most cases, two surfaces with different characteristics may be referred to as a Janus particle. The Janus particle anisotropically interacts with an environment such as other molecules, and therefore, in a dispersion system for example, it is self-assembled into a wide variety of structures depending on different regions, and a crystal structure not found in a normal isotropic particle is formed. Accordingly, the Janus particle has also been used for a model system for elucidating characteristics of dispersion properties of an anisotropic particle and physical properties such as dynamical behavior of the particle.

The present inventors have developed methods of easily producing a Janus particle having hemispheres, each of which is composed of a different material (Patent Literatures 1 and 2). These methods use a self-organized precipitation (SORP) method in which, to a solution containing two polymers having different characteristics, a poor solvent in which those polymers are not dissolved is added, and then, the good solvent is evaporated away to replace it with the poor solvent, thereby obtaining a dispersion containing phase-separated, submicron-sized polymer fine particles.

### Citation List

### Patent Literature

Patent Literature 1: JP Patent No. 5103611
Patent Literature 2: JP Patent No. 5239004

### Summary of Invention

### Technical Problem

Conventionally, an ELISA (Enzyme-Linked Immuno-Sorbent Assay) method, radioimmunoassay or the like has been generally used for detecting a specimen substance having a biological origin, but the operation process is complicated and also time-consuming. Therefore, improvement has been demanded.

### Solution to Problem

The present inventors have focused on that the already-developed Janus particle that can be prepared by the self-organized precipitation method can be utilized for improving the above-described detection method for a specimen substance, and proceeded with various investigations for configuration and practical application of the fine particle. As a result, a Janus particle functionalized for detecting a specimen substance has been successfully obtained by modifying one surface of the fine particle with the specimen substance or a substance that can specifically bind to the specimen substance and by modifying the other surface with a labeling substance for detection. Furthermore, by mixing a magnetic material into a polymer solution during the course of producing the Janus particle, a magnetic Janus particle having magnetic field responsiveness has been successfully made.

Meanwhile, the present inventors have developed a chip device that can be fixed to the inside of a microchannel (for example, JP Patent Publication (Kokai) No. 2008-014791 A (2008)). With this chip device, a capturing reagent that interacts with a specimen substance can be immobilized on the surface within the channel, and therefore, a variety of specimen substances can be detected.

By combining the functionalized Janus particle with the chip device, a measuring sensor is obtained that can function for quick and easy detection of a specimen substance with higher accuracy than that of conventional detection methods.

That is, the present invention is defined in the appended claims.

The present application claims priority to JP Patent Application No. 2017-084812

### Advantageous Effects of Invention

The measuring sensor obtained by combining the multiphase polymer fine particle according to the present invention and the chip device can improve the sensitivity and accuracy of a detection system for a variety of specimen substances having biological origin or the like by utilizing characteristics of dispersion properties of the anisotropic Janus particle and physical properties such as dynamical behavior of the particle, and can be utilized for, for example, diagnosis with disease biomarkers, or examination for virus or pathogenic organism. Brief Description of Drawings

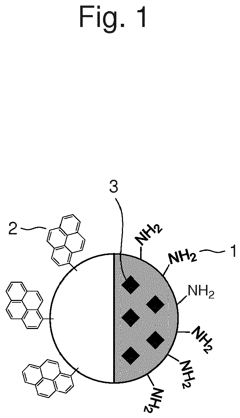

[Figure 1] Figure 1 schematically illustrates a multiphase polymer fine particle that can be used for the present invention.

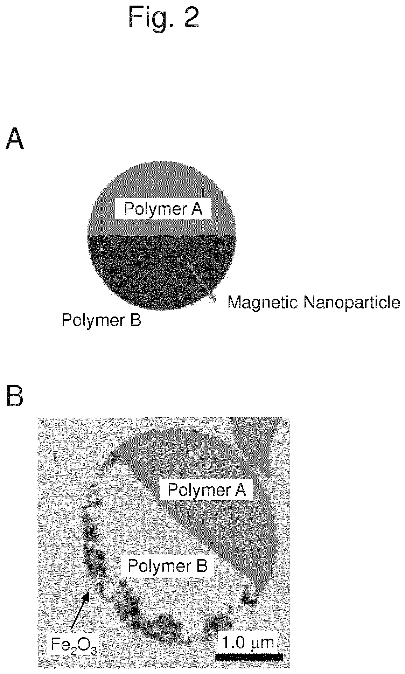

[Figure 2] Figure 2A schematically illustrates an example in which magnetic nanoparticles are incorporated in a part of polymer phases (polymer B) in the multiphase polymer fine particle.
Figure 2B illustrates an electron micrograph of the multiphase polymer fine particle in which iron oxide (Fe₂O₃) is incorporated in polymer B.

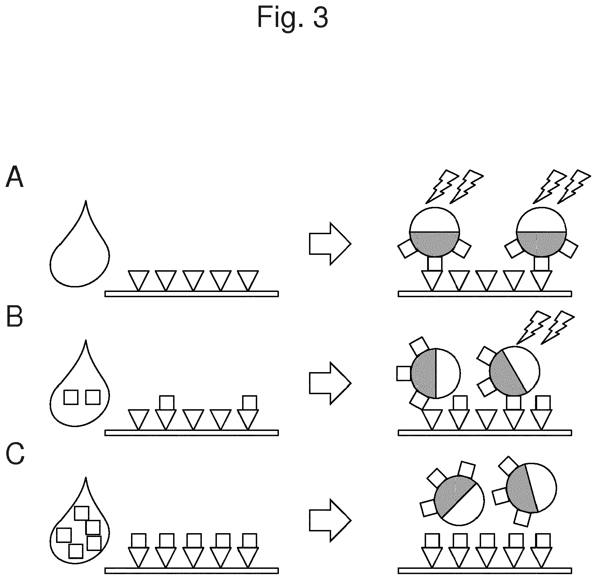

[Figure 3] Figure 3 schematically illustrates one embodiment of a detection method using the multiphase polymer fine particle according to the present invention. A: When the specimen substance (□) is not contained in a sample, a high signal (for example, fluorescence) intensity is detected from a labeling substance on the multiphase polymer fine particle that has bound to the capturing reagent (∇). B and C: When the specimen substance is contained in a sample, the amount of the multiphase polymer fine particle that has bound to the capturing reagent becomes smaller, and a signal (for example, fluorescence) intensity to be detected becomes lower.

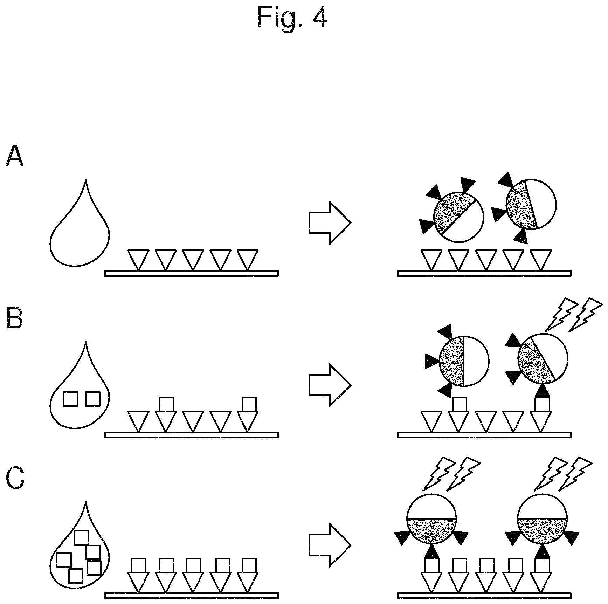

[Figure 4] Figure 4 schematically illustrates one embodiment of a detection method using the multiphase polymer fine particle according to the present invention. A: When the specimen substance (□) is not contained in a sample, the multiphase polymer fine particle does not bind to the capturing reagent (V), and therefore, a signal (for example, fluorescence) is not detected from the multiphase polymer fine particle. B and C: When the specimen substance is contained in a sample, the substance that can specifically bind to the specimen substance (**▼**) on the multiphase polymer fine particle binds to the specimen substance that has bound to the capturing reagent, and a signal (for example, fluorescence) is detected from a labeling substance.

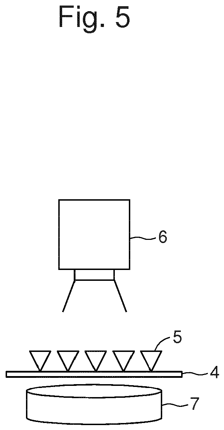

[Figure 5] Figure 5 schematically illustrates a system according to the present invention.

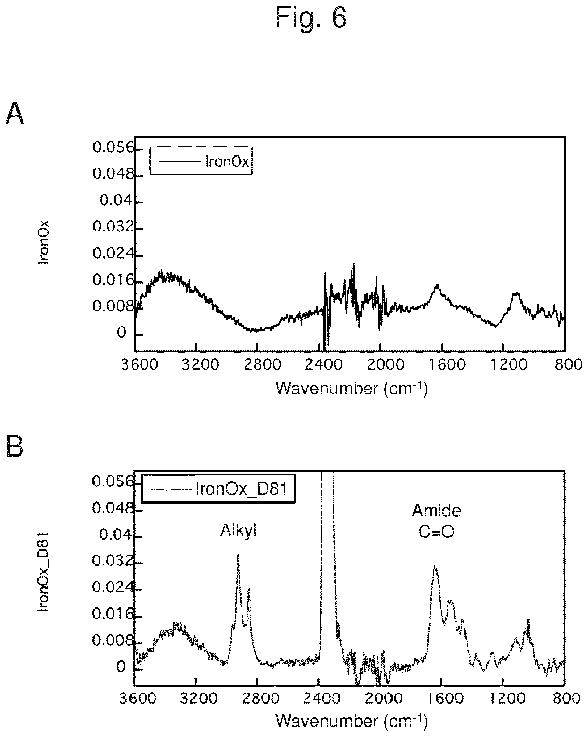

[Figure 6] Figure 6 illustrates a measurement result by a Fourier transform infrared spectrophotometer (FT-IR). A: Iron oxide (Fe₂O₃) before the reaction with D81. B: magnetic nanoparticle covered with D81 after the reaction.

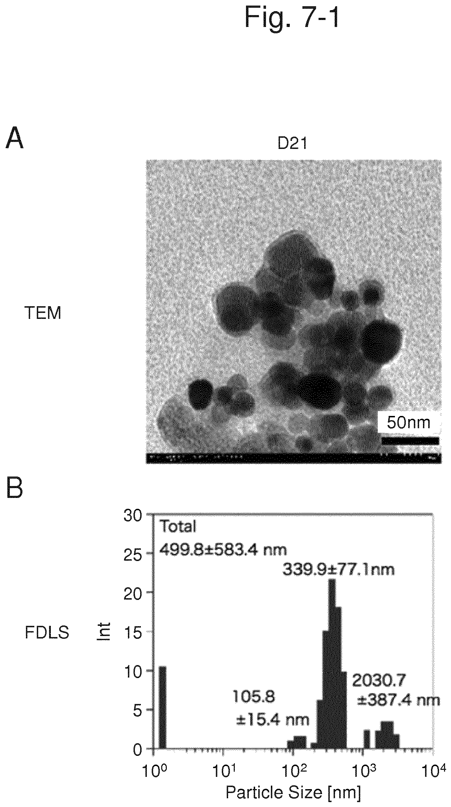

[Figure 7-1] Figure 7-1 illustrates a photograph by a transmission electron microscope (TEM) (A) and the particle size distribution by a fiber-optic dynamic light scattering (FDLS) photometer (B), of magnetic nanoparticles covered with 2:1 random copolymer of dodecylacrylamide and dopamine methacrylamide (D21).

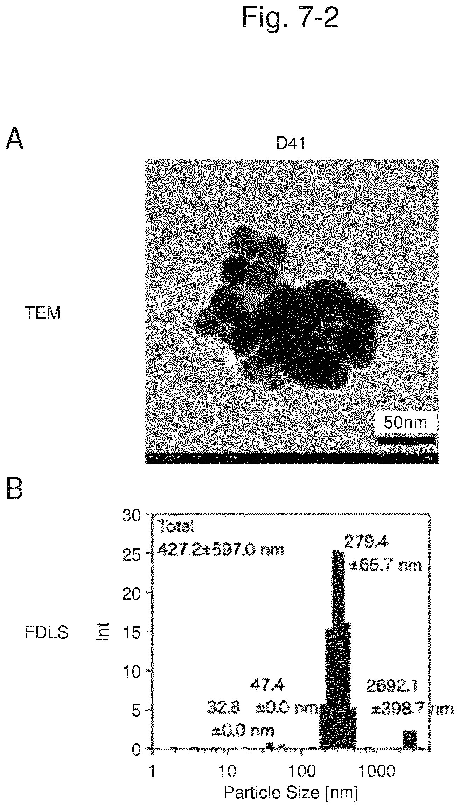

[Figure 7-2] Figure 7-2 illustrates a photograph by a transmission electron microscope (TEM) (A) and the particle size distribution by a fiber-optic dynamic light scattering (FDLS) (B) photometer, of magnetic nanoparticles covered with 4:1 random copolymer of dodecylacrylamide and dopamine methacrylamide (D41).

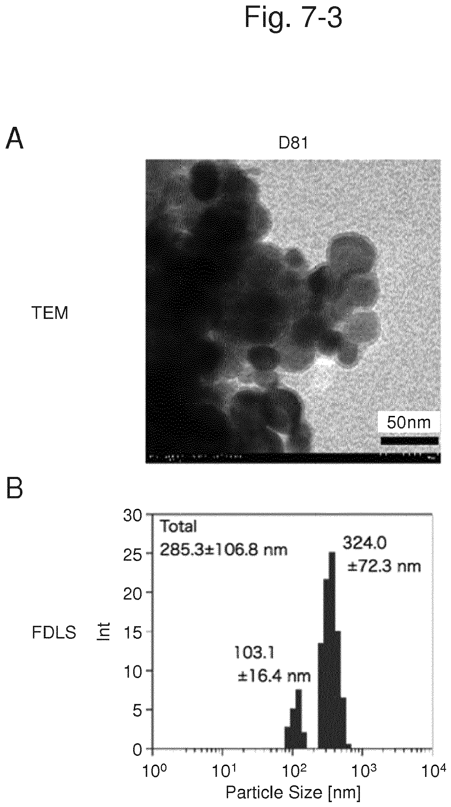

[Figure 7-3] Figure 7-3 illustrates a photograph by a transmission electron microscope (TEM) (A) and the particle size distribution by a fiber-optic dynamic light scattering (FDLS) (B) photometer, of magnetic nanoparticles covered with 8:1 random copolymer of dodecylacrylamide and dopamine methacrylamide (D81).

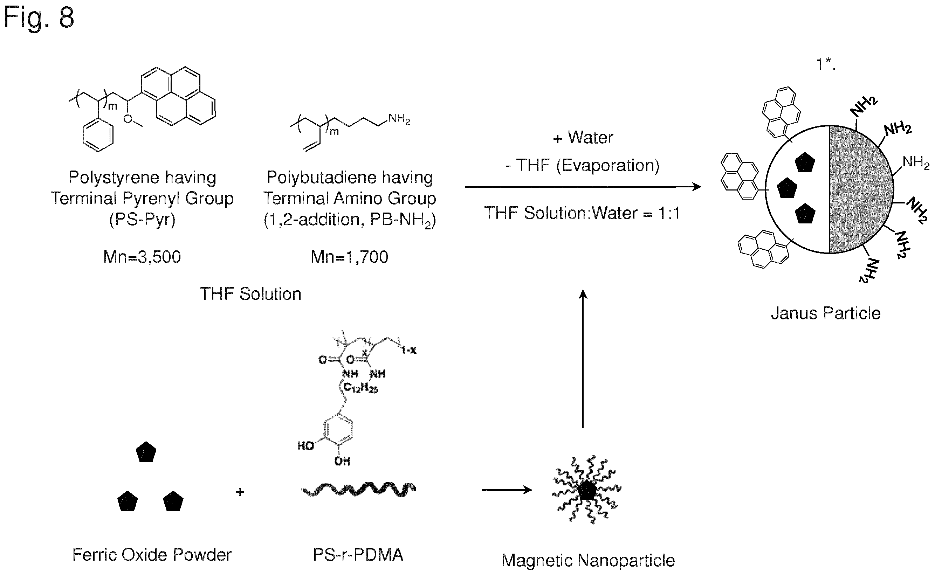

[Figure 8] Figure 8 illustrates a production process of a multiphase polymer fine particle according to the present invention.

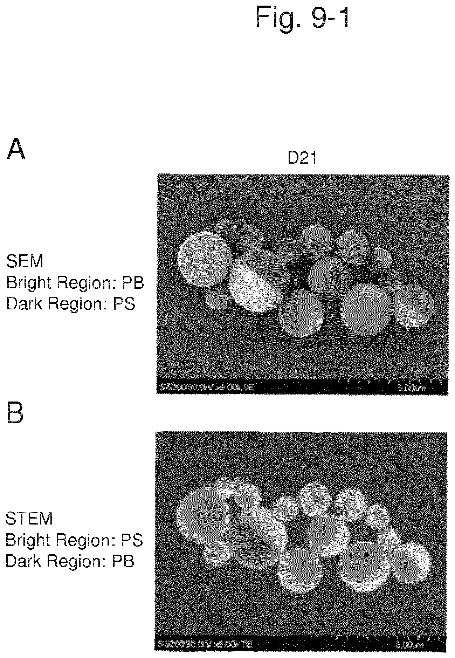

[Figure 9-1] Figure 9-1 illustrates an electron micrograph of a multiphase polymer fine particle according to the present invention, in which magnetic nanoparticles covered with D21 are incorporated. A: A scanning electron microscope image, in which the polybutadiene (PB) region is shown bright and the polystyrene (PS) region is shown dark. B: A scanning transmission electron microscope image, in which the polybutadiene (PB) region is shown dark and the polystyrene (PS) region is shown bright.

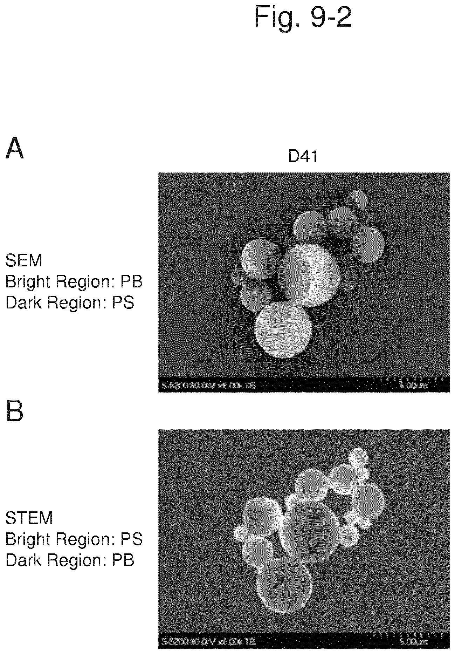

[Figure 9-2] Figure 9-2 illustrates an electron micrograph of a multiphase polymer fine particle according to the present invention, in which magnetic nanoparticles covered with D41 are incorporated. A: A scanning electron microscope image, in which the polybutadiene (PB) region is shown bright and the polystyrene (PS) region is shown dark. B: A scanning transmission electron microscope image, in which the polybutadiene (PB) region is shown dark and the polystyrene (PS) region is shown bright.

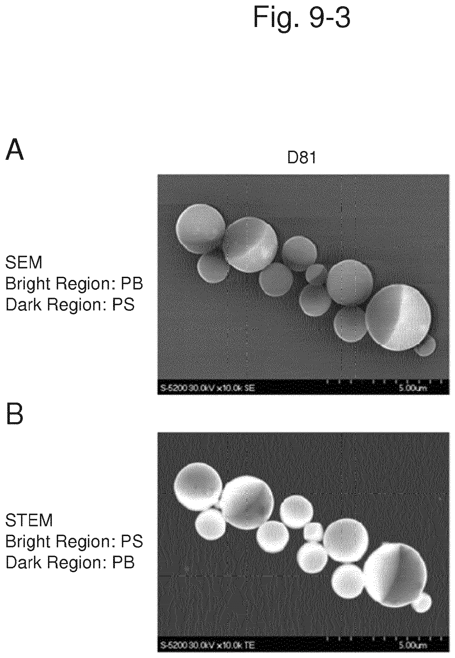

[Figure 9-3] Figure 9-3 illustrates an electron micrograph of a multiphase polymer fine particle according to the present invention, in which magnetic nanoparticles covered with D81 are incorporated. A: A scanning electron microscope image, in which the polybutadiene (PB) region is shown bright and the polystyrene (PS) region is shown dark. B: A scanning transmission electron microscope image, in which the polybutadiene (PB) region is shown dark and the polystyrene (PS) region is shown bright.

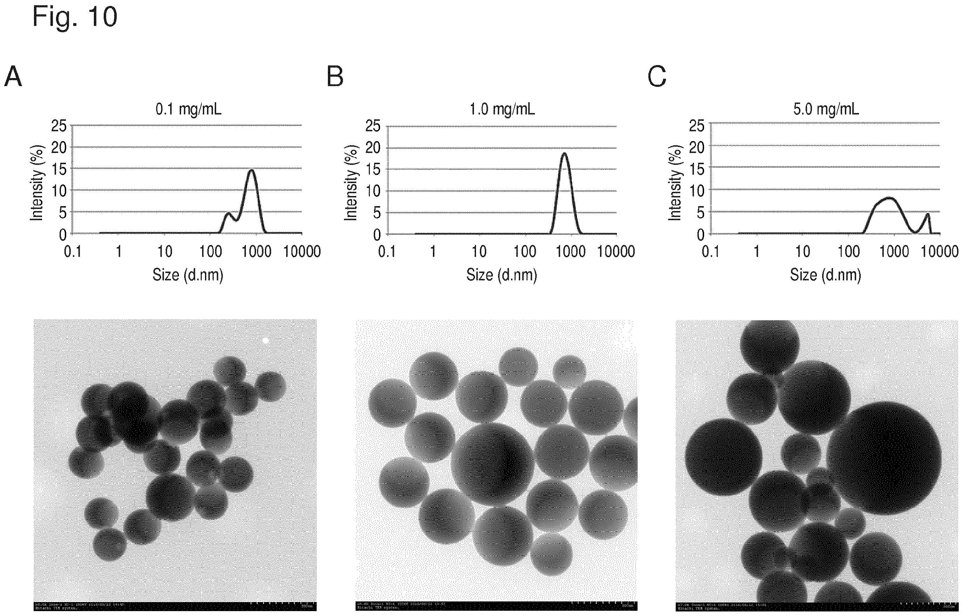

[Figure 10] Figure 10 illustrates the particle size of multiphase polymer fine particles prepared by using THF solutions of polystyrene and polybutadiene at a concentration of 0.1 mg/mL (A), 1.0 mg/mL (B) or 5.0 mg/mL (C), with graphs and micrographs.

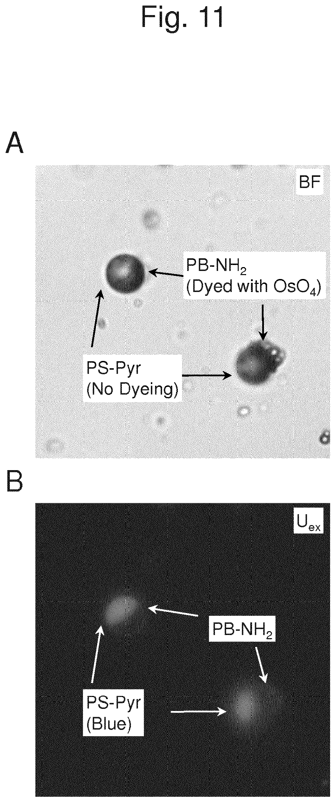

[Figure 11] Figure 11 illustrates that a part of polymer phases in a multiphase polymer fine particle is selectively labeled. A: In the bright field image, a polymer region dyed with osmium tetroxide (polybutadiene) and another polymer region with no dyeing (polystyrene) are shown. B: In the fluorescence image, blue fluorescence can be seen in the region composed of polystyrene and no fluorescence can be seen in the region composed of polybutadiene.

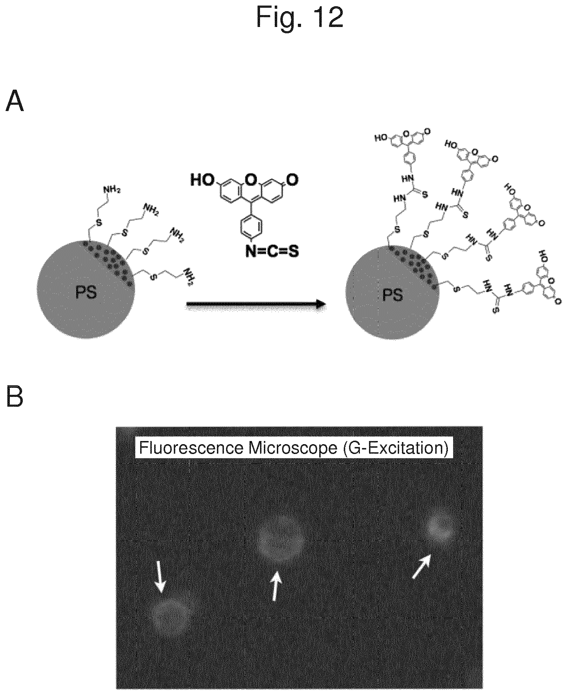

[Figure 12] Figure 12A illustrates an example in which a fluorescent dye modifies a half surface of a magnetic nanoparticle. Figure 12B illustrates a fluorescence microscope image, in which fluorescence is observed only in a part of polymer phases.

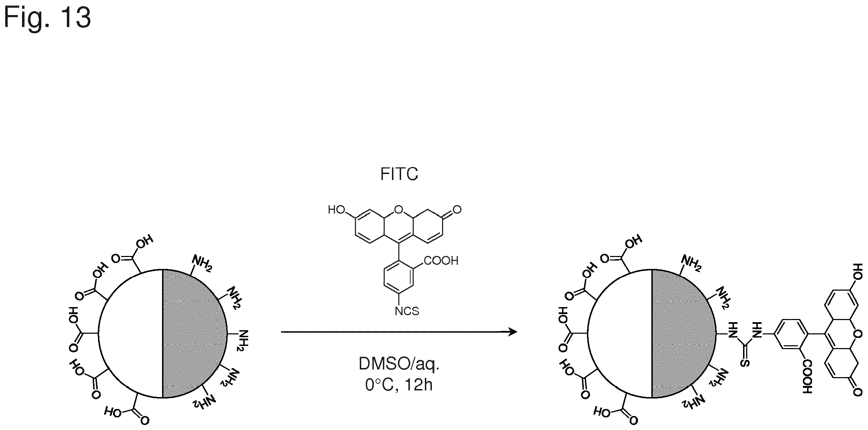

[Figure 13] Figure 13 illustrates an example in which a fluorescent dye modifies a half surface of a non-magnetic nanoparticle.

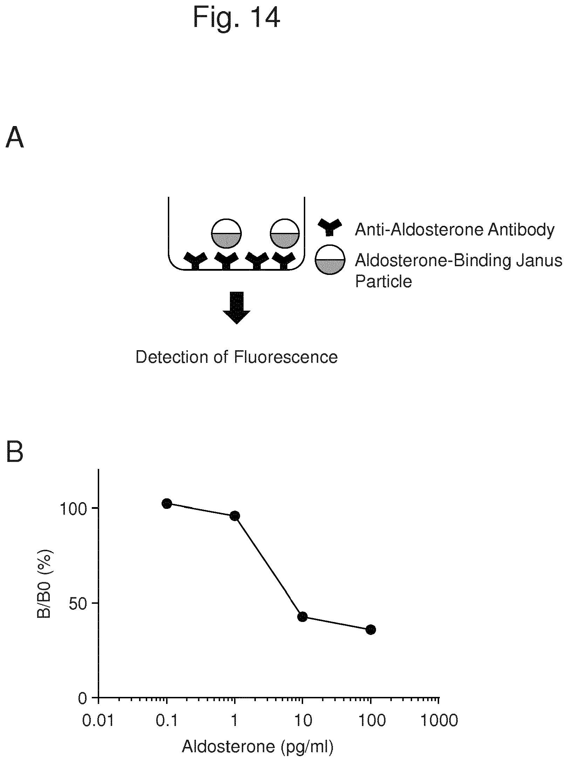

[Figure 14] Figure 14 illustrates detection of aldosterone by the method according to the present invention. A: The detection method is schematically shown. B: The fluorescent intensity declines depending on an increase in the concentration of aldosterone in a sample. The vertical axis shows a relative fluorescent intensity (%) where the fluorescent intensity when no aldosterone is contained (B0) is defined as 100, and the horizontal axis shows the concentration of aldosterone in the sample (pg/mL).

### Description of Embodiments

### [Multiphase Polymer Fine Particle]

The present invention provides a multiphase polymer fine particle having at least on a surface thereof two or more polymer phases formed by aggregation of two or more polymers, respectively, wherein a first polymer phase can bind to a specimen substance or a substance that can specifically bind to the specimen substance, and wherein a second polymer phase has a labeling substance. It is only required for the fine particle to include two or more polymer phases, and it may include three, or four or more polymer phases, as necessary. However, the smaller number of polymer phases makes production of the fine particle easier, and therefore, the number of polymer phases is only required to be two for the purpose of the present invention, that is, to detect the specimen substance in a sample. For distinguishing polymer phases, they are described in the present specification as a "first polymer phase", a "second polymer phase", or the like for convenience sake, but these notations are not to specify the types of polymers.

For two or more polymers constituting the multiphase polymer fine particle according to the present invention, either of them can be selected from the following polymers:
1) water-soluble polymers such as polymers of N-isopropylacrylamide, polyethylene glycol, and the like;
2) water-insoluble polymers such as 1,4-cis-isoprene, isoprene elastomer, polystyrene, polybutadiene, polyisoprene, polymethyl methacrylate, poly(n-butyl acrylate), polyvinyl chloride, polyacrylonitrile and polylactic acid; and
3) copolymers such as butadiene-styrene copolymer.

The multiphase polymer fine particle having two or more polymer phases formed by aggregation of two or more polymers, respectively, can be produced by, for example, methods described in JP Patent No. 5103611 and JP Patent No. 5239004 previously invented by the present inventors. In these methods, as the above-described two or more polymers, polymers with the difference in solubility parameter within a particular range are selected, and these polymers are dissolved in a good solvent therefor to prepare a good solvent solution. Thereafter, a solvent, which is compatible with the good solvent but is a poor solvent for these polymers, is added to the solution, and the good solvent is evaporated away, thereby forming a fine particle having a plurality of polymer phases formed by separate aggregation of the polymers, respectively. Similarly, when three or more polymer phases are present, three or more polymers are dissolved in a commonly good solvent therefor to prepare a good solvent solution. Thereafter, a solvent, which is compatible with the good solvent but is a commonly poor solvent for these three or more polymers, is admixed with the solution, and the good solvent is then evaporated, thereby forming a fine particle having three or more polymer phases.

For example, when two polymers are used, examples of suitable combination include, but are not limited to, polyethylene glycol and N-isopropylacrylamide, polystyrene and polyisoprene, polystyrene and polybutadiene, polystyrene and polylactic acid, and polystyrene and polybutyl acrylate. In addition, when the combination of polyethylene glycol and N-isopropylacrylamide is used, water can be used as the good solvent, and DMSO or 1 -propanol can be used as the poor solvent. When the combination of polystyrene and polyisoprene is used, tetrahydrofuran (THF) can be used as the good solvent, and water can be used as the poor solvent. Depending on the types and combination of polymers to be used, the good solvent and the poor solvent that can be used for preparing a fine particle can be selected properly.

A production method for the multiphase polymer fine particle according to the present invention is not limited to the method described above, and it may be in the form in which, for example, a core of silica, ceramic or the like that has been made in advance is covered with two or more polymer phases having high affinity with the core. That is, the multiphase polymer fine particle is not necessarily a fine particle, the entirety of which is formed of polymers, and it is only required to be a fine particle having two or more polymer phases at least on a surface thereof, using techniques normally used in the art.

The multiphase polymer fine particle according to the present invention is configured such that the first polymer phase can bind to a specimen substance or a substance that can specifically bind to the specimen substance. In the present specification, the "specimen substance" refers to an already-known substance that may be contained in a sample such as a biological sample, and the presence or amount of which is required to be detected. Examples of the specimen substance may include, but are not particularly limited to, a natural biological substance and an agent administered for treatment, prophylaxis or diagnosis of a disease, such as a protein or peptide, a saccharide, a nucleic acid, a lipid or a steroidal compound.

In the present specification, the "substance that can specifically bind to the specimen substance" may be any substance as long as it can specifically bind to the specimen substance, and examples thereof include, but are not particularly limited to, an antibody, a receptor and an aptamer. Alternatively, when the specimen substance is a nucleic acid, the substance may be a nucleic acid (for example, DNA) that has a sequence complementary to the nucleic acid sequence of the specimen substance. When the specimen substance is a saccharide, the substance may be a lectin. The substance that can specifically bind to a specimen substance is preferably an antibody. The specimen substance or the substance that can specifically bind to the specimen substance, covalently binding to the first polymer phase, has a molecular weight in the range of 100 to 1 million and a size in the range of 1 nm to 100 nm, for example.

The antibody may be a natural antibody derived from an arbitrary biological species, a genetically modified antibody, or a synthesized antibody. The antibody that is allowed to covalently bind to the first polymer phase may be a polyclonal antibody or a monoclonal antibody as long as it can specifically bind to the specimen substance, and therefore, a mixture of a plurality of antibodies having different binding specificity can also be used for the binding. The antibody may be, but is not particularly limited to, an IgG antibody that can specifically bind to the specimen substance of interest, a fragment or a derivative thereof retaining the antigen binding ability. For example, a Fab fragment, or a F(ab')₂ fragment can be suitably used. Moreover, the antibody may have a modification as normally used in the art, for example, for the purpose of enhancing structural stability or for the subsequent detection step.

In order to ensure that the specimen substance or the substance that can specifically bind to the specimen substance, binding to the first polymer phase, exhibits common behavior with the multiphase polymer fine particle, the binding between the first polymer phase and the specimen substance or the substance that can specifically bind to the specimen substance is preferably a covalent bonding. In order to enable binding with the specimen substance or the substance that can specifically bind to the specimen substance, a polymer having a functional group such as an amino group or a carboxyl group at the terminus can be used as the polymer constituting the first polymer phase. The polymer having an amino group or a carboxyl group at the terminus can be synthesized, or can be purchased as a commercial product from, for example, Polymer Source. Inc. In this case, an amino group or a carboxyl group is present on the first polymer phase of the produced multiphase polymer fine particle, and therefore, the group can form a covalent bonding with a carboxyl group, a cyano group, an amino group or the like in the specimen substance or the substance that can specifically bind to the specimen substance.

The number of molecules of the specimen substance or the substance that can specifically bind to the specimen substance, binding to one multiphase polymer fine particle may be in the range of 1 to 1000. The amount of the specimen substance or the substance that can specifically bind to the specimen substance per one fine particle can be appropriately adjusted by selecting reaction conditions for generating the above-described covalent bonding, and it is variable depending on the application. For example, when the specimen substance is detected by utilizing competitive binding (the same applies to indirect competitive binding) between the specimen substance in the sample and the specimen substance covalently bound to the fine particle, the number of molecules of the specimen substance covalently bound to the fine particle may be 10 or less, preferably 5 or less, and particularly preferably 1 per fine particle. When the specimen substance is detected utilizing a binding between the specimen substance in the sample and the substance that can specifically bind to the specimen substance covalently binding to the fine particle, the number of molecules of the substance that can specifically bind to the specimen substance, covalently binding to the fine particle may be, but not particularly limited to, 10 or less, or 5 or less, such as 1, per fine particle.

The multiphase polymer fine particle according to the present invention is configured such that the second polymer phase has a labeling substance. Examples of the labeling substance include a fluorescent dye, a chemiluminescent substance and a radioactive labeling substance. Examples of the fluorescent dye include, but are not particularly limited to, fluorescent dyes with an excellent quantum yield, such as fluorescein and a derivative thereof, pyrene and a derivative thereof, and a quantum dot. Examples of the chemiluminescent substance include enzymes such as peroxidase (HRP) and alkaline phosphatase (ALP). Examples of the radioactive labeling substance include reagents having ¹⁴C, ³H and ¹²⁵I. In order to enable binding with the labeling substance, for the polymer constituting the second polymer phase, for example, a polymer having a functional group such as an amino group or a carboxyl group at the terminus can be used. The polymer having an amino group or a carboxyl group at the terminus can be synthesized, or alternatively, it can be purchased as a commercial product from, for example, Polymer Source. Inc. In this case, an amino group or a carboxyl group is present on the second polymer phase of the produced multiphase polymer fine particle, and therefore, the group can form a covalent bonding with a carboxyl group or a cyano group, an amino group or the like in the labeling substance. Alternatively, for the second polymer phase, a polymer having a pyrenyl group or the like at the terminus, a polymer modified to have a chemiluminescent substance, or a polymer modified to have a radioactive isotope can also be used. In this case, the labeling substance may be present on the second polymer phase upon producing the multiphase polymer fine particle.

The first and second polymer phases can be appropriately selected. When the labeling substance is allowed to bind to the second polymer phase on the produced multiphase polymer fine particle, functional groups produced on the polymer phases, such as an amino group and a carboxyl group, need to be different such that the specimen substance or the substance that can specifically bind to the specimen substance and the labeling substance can bind to the first polymer phase and the second polymer phase, respectively.

The ratio between the first and the second polymer phases is not particularly limited, and may be in the range of 1:9 to 9:1 in terms of their respective surface areas on the fine particle. The ratio between polymer phases can be appropriately changed by adjusting the ratio between the amounts of polymers used upon producing the fine particles.

Note that the term "Janus particle" is generally used for an asymmetric spherical particle having two hemispheres that are physically and/or chemically different, and the multiphase polymer fine particle described in the present specification may also be an asymmetric "Janus particle" having anisotropy.

The multiphase polymer fine particle according to the present invention can contain a magnetic material in a part of polymer phases. A polymer phase containing the magnetic material may be either of the above-described first or second polymer phase, or it may be a third polymer phase different from them. By incorporating the magnetic material in a polymer phase, behavior of the multiphase polymer fine particle can be controlled by the magnetic field.

Examples of the magnetic material include, but are not particularly limited to, iron, chromium, cobalt, neodymium, and oxides and sulfides thereof, and they can be suitably used. The size of the magnetic material is not particularly limited, and in order to incorporate it in the nano-sized fine particle, it can be a magnetic nanoparticle with a particle size in the range of 1 to 100 nm. In addition, several to dozens of those magnetic nanoparticles may form an aggregate. For the magnetic nanoparticle, for example, iron oxide nanoparticles manufactured by Sigma-Aldrich Co. LLC can be suitably used.

A multiphase polymer fine particle that can be used for the present invention is schematically illustrated in Figure 1. The fine particle illustrated in Figure 1 has an amino group 1 on one polymer phase (a first polymer phase), and can covalently bind to the specimen substance or a substance that can specifically bind to the specimen substance via this amino group. The other polymer phase may have a labeling substance, for example, a fluorescent substance 2. In Figure 1, a magnetic material 3 is incorporated in the first polymer phase.

Figure 2A schematically illustrates an example in which magnetic nanoparticles are incorporated in polymer B phase in a multiphase polymer fine particle composed of polymer A and polymer B. Figure 2B illustrates an electron micrograph of a multiphase polymer fine particle in which iron oxide (Fe₂O₃) is incorporated in polymer B. The polymer A and the polymer B may be either of the above-described first or second polymer phase.

In order to stably incorporate the magnetic material in the polymer, a compound having a part with affinity to the magnetic material and a part with affinity to the polymer, for example, polystyrene-polydopamine methacrylamide random copolymer (PS-r-PDMA) may be used, and the magnetic material can be incorporated in the multiphase polymer fine particle in a state where it is covered with a material having affinity to the polymer. The magnetic material, the affinity to the polymer of which has been increased as such, can be admixed in a polymer solution during the course of producing the multiphase polymer fine particle, thereby incorporating it in a precipitated fine particle. The exemplified PS-r-PDMA includes polystyrene, and therefore, can be conveniently incorporated in a polymer phase composed of polystyrene polymer. Coverage of the magnetic material with a polymer can be suitably carried out by, for example, the method described in JP No. 5008009. The magnetic material is suitably incorporated such that the multiphase polymer fine particle to be obtained can be collected in a magnetic field of, for example, about 50 mT or more.

The multiphase polymer fine particle according to the present invention can have a functional group on the surface of the first polymer phase as described above, and can covalently bind to the specimen substance or the substance that can specifically bind to the specimen substance, having a group reactive with the functional group. When the specimen substance or the substance that can specifically bind to the specimen substance itself has a reactive group, it can covalently bind to the multiphase polymer fine particle directly. When the specimen substance or the substance that can specifically bind to the specimen substance does not have a reactive group, it can be chemically modified in advance such that the specimen substance or the substance that can specifically bind to the specimen substance has a reactive group. In this case, in order not to hinder a binding between a capturing reagent, which will be described later, and the specimen substance, or a binding between the substance that can specifically bind to the specimen substance and the specimen substance, a reactive group or a modification site needs to be selected with consideration. For example, when the specimen substance is an antigen and the capturing reagent is an antibody, a site other than the epitope on the antigen, which the antibody specifically recognizes and binds to, needs to be subjected to binding with the fine particle or modification for the binding. Those having ordinary skill in the art can appropriately select and determine a reactive group and a modification site depending on the type of the specimen substance or the substance that can specifically bind to the specimen substance and the type of binding with the capturing reagent. A linker normally used in the art may be used for the binding.

When a protein such as an antibody is allowed to bind to the multiphase polymer fine particle, numerous amino groups and carboxyl groups are included in the constituent amino acids of the protein, and therefore, the binding with the multiphase polymer fine particle can be achieved by mixing them in the presence of a condensing agent. When an antibody is allowed to bind to the multiphase polymer fine particle, it is necessary to use an antibody that recognizes a site other than the site of the specimen substance to which the capturing reagent binds.

In one aspect, the multiphase polymer fine particle according to the present invention can have a functional group on the surface of the second polymer phase as described above, and can covalently bind to a labeling substance having a reactive group that reacts with the functional group. Similarly as the specimen substance, when the labeling substance itself has a reactive group, it can covalently bind to the multiphase polymer fine particle directly, and when the labeling substance does not have a reactive group, it can be chemically modified in advance such that the labeling substance has a reactive group. Alternatively, in another aspect, the labeling substance can be incorporated in the second polymer phase during the course of producing the polymer fine particle, as described above.

It is possible to obtain the multiphase polymer fine particle with an average particle size in the range of 1 nm to 1 mm, for example, 100 nm to 1 mm, depending on production conditions. For example, with no particular limitation, the multiphase polymer fine particle can have an average particle size in the range of about 10 nm to about 100 µm, preferably about 50 nm to about 50 µm, and further preferably about 100 nm to about 10 µm, for detecting the specimen substance in a small device as one application. If a magnetic material is enclosed in the polymer phase, in order to enclose a sufficient amount of the magnetic material, the multiphase polymer fine particle may suitably have an average particle size of about 100 nm or more. The coefficient of variation in the particle size is within 20%, and preferably within 10%.

The multiphase polymer fine particle according to the present invention can be used to measure the specimen substance in a sample with high sensitivity and high accuracy by the method specifically described in the following.

### [Method of Detecting Specimen Substance]

The present invention also provides a method of detecting the specimen substance in a sample. The method according to the present invention comprises the following steps:
a step of contacting a sample with a solid phase support onto which capturing reagents that specifically bind to the specimen substance are immobilized;
a step of contacting the multiphase polymer fine particle according to the present invention with the solid phase support; and
a step of detecting a label on the fine particle bound on the solid phase support.

In one embodiment of the method according to the present invention, the fine particle is a fine particle to which the specimen substance has covalently bound. The specimen substance in the sample and the specimen substance present on the fine particle competitively bind to the capturing reagent, and a capturing reagent not bound to the specimen substance in the sample binds to the specimen substance present on the fine particle.

In another embodiment of the method according to the present invention, the fine particle is a fine particle to which the substance that can specifically bind to the specimen substance has covalently bound, and the specimen substance in the sample bound to the capturing reagent binds to the substance that can specifically bind to the specimen substance present on the fine particle.

In the method described above, examples of the sample include, but are not particularly limited to, a biological sample that may contain a specimen substance, such as blood (including plasma, and serum), cerebrospinal fluid, pus, puncture fluid, urine and stool, and in most cases, the sample is blood, plasma or serum. The sample may be used for the method according to the present invention as it is. Alternatively, before the step of contacting the sample with the solid phase support, a step of lysis and/or a step of removing contaminant substances that are unrelated to the reaction, such as erythrocyte, leukocyte, platelet and proteins other than the detection object may be included. The step of removing contaminant substances can be carried out by the operation of, for example, centrifugation. In addition, the sample may be diluted or concentrated, as necessary. The lysis and dilution can be carried out using an aqueous solution suitable for the method according to the present invention, for example, phosphate buffered saline.

The capturing reagent may be, but is not particularly limited to, an antibody against the specimen substance as an antigen, a lectin that can specifically bind to a saccharide, or a polynucleotide that has complementarity allowing hybridization with a nucleic acid substance. The antibody may be a natural antibody derived from an arbitrary biological species, a genetically modified antibody, or a synthesized antibody. The antibody may be, but is not particularly limited to, an IgG antibody that can specifically bind to the specimen substance of interest, a fragment or a derivative thereof retaining the antigen binding ability. For example, a Fab fragment, or a F(ab')₂ fragment can be suitably used. In addition, the polynucleotide may be DNA, RNA or a synthesized polynucleotide that has been modified to improve stability or the like. The capturing reagent and the specimen substance preferably bind to each other at 1:1 in terms of molecules.

It is desired that the amount of the capturing reagent be the same as or more than the expected amount of the specimen substance in the sample, in terms of molecules. This is because when the amount of the capturing reagent is too small, a part of the specimen substance to be detected does not bind to the capturing reagent, and thus, it is not possible to accurately detect the specimen substance. In the subsequent steps, the multiphase polymer fine particle is allowed to bind to the solid phase support and a label of the multiphase polymer fine particle bound to the solid phase support is detected. As such, it is preferable that the amount of the capturing reagent be excessive to, for example, 1.1 times or more, 1.2 times or more, 1.5 times or more, 2 times or more, or 3 times or more the expected amount of the specimen substance in the sample.

The capturing reagent is immobilized onto the solid phase support for the subsequent steps. For the solid phase support, a material normally used in the art, such as plastic, glass and silicon can be suitably used. For the solid phase support, those having been subjected to surface treatment can be used in order to prevent nonspecific adsorption of the specimen substance.

The contact between the sample and the solid phase support may be carried out in the static state or fluidly. For example, the sample may be added onto the solid phase support such as a well to be brought into contact therewith, or alternatively, the sample may be passed through a channel to which the solid phase support is fixed for the contact. After contacting the sample with the solid phase support, components other than the specimen substance in the sample are preferably removed by washing with a buffer solution.

Next, the multiphase polymer fine particle according to the present invention is contacted with the solid phase support to which the specimen substance in the sample has bound. As described above, the amount of the capturing reagent on the solid phase support is the same as or more than the amount of the specimen substance in the sample, in terms of molecules, and is preferably excessive thereto. Accordingly, on the solid phase support, there may be the capturing reagent not bound to the specimen substance in the sample.

In an embodiment where a fine particle to which a specimen substance has covalently bound is used as the multiphase polymer fine particle, substantially the same specimen substance as the specimen substance in the sample has covalently bound to the multiphase polymer fine particle, and therefore, the specimen substance in the state of binding to the multiphase polymer fine particle can bind to the capturing reagent. That is, the specimen substance that has covalently bound to the multiphase polymer fine particle and the specimen substance in the sample have substantially the same structure, and are capable of competitively binding to the capturing reagent, and have equivalent binding affinity to the capturing reagent.

In this embodiment, the sample is first contacted with the capturing reagent, and therefore, the specimen substance that has covalently bound to the multiphase polymer fine particle can bind to an unbound capturing reagent without inhibiting the binding of the specimen substance that has already bound to the capturing reagent. The fine particle that has not bound to the capturing reagent can be removed by a subsequent washing operation, as necessary.

In this embodiment, a label on the fine particle that has bound to the capturing reagent on the solid phase support is then detected. The label is, for example, a fluorescent dye, a chemiluminescent substance or a radioactive labeling substance, and the label on the fine particle can be detected by detecting fluorescence, chemiluminescence or radiation from the radioactive substance, respectively.

As illustrated in Figure 3, when the sample contains a large amount of the specimen substance (Figure 3C), the specimen substance (indicated by □) binds to the capturing reagent (indicated by V), thereby reducing the amount of an unbound capturing reagent. Accordingly, the binding between the capturing reagent and the specimen substance (□) on the first polymer phase of the multiphase polymer fine particle is competitively inhibited, and therefore, the detection intensity of fluorescence or the like from the labeling substance on the second polymer phase of the fine particle bound to the capturing reagent becomes lower.

In contrast, when the sample does not contain the specimen substance (Figure 3A) or contains only a small amount of the specimen substance (Figure 3B), the capturing reagent, that has not bound to the specimen substance, binds to the specimen substance on the first polymer phase of the multiphase polymer fine particle, and therefore, the detection intensity of fluorescence or the like from the labeling substance on the second polymer phase of the fine particle is higher.

In an embodiment where a fine particle to which a substance that can specifically bind to a specimen substance has covalently bound is used as the multiphase polymer fine particle, the substance that can specifically bind to a specimen substance in the state of binding to the multiphase polymer fine particle can bind to the specimen substance in the sample that has bound to the capturing reagent. In this case, the substance that can specifically bind to the specimen substance, binding to the multiphase polymer fine particle and the capturing reagent recognize and bind to different sites in the specimen substance, and the detection method is a sandwich assay method.

In this embodiment, the sample is first contacted with the capturing reagent, and therefore, the substance that can specifically bind to the specimen substance, covalently binding to the multiphase polymer fine particle binds to the specimen substance that has already bound to the capturing reagent, and does not react with a capturing reagent to which the specimen substance has not bound. The fine particle that has not bound to the specimen substance can be removed by a subsequent washing operation, as necessary.

In this embodiment, a label on the fine particle that has bound to the specimen substance present on the solid phase support via the capturing reagent is then detected. The label is, for example, a fluorescent dye, a chemiluminescent substance or a radioactive labeling substance, and the label on the fine particle can be detected by detecting fluorescence, chemiluminescence or radiation from the radioactive substance, respectively.

As illustrated in Figure 4, when the sample contains a large amount of the specimen substance (Figure 4C), the specimen substance (indicated by □) binds to the capturing reagent (indicated by V), and more substances that can specifically bind to the specimen substance on the first polymer phase of the multiphase polymer fine particle (A) bind to the specimen substance. Therefore, the detection intensity of fluorescence or the like from the labeling substance on the second polymer phase of the fine particle that has bound to the specimen substance becomes higher.

In contrast, when the sample does not contain the specimen substance (Figure 4A) or contains only a small amount of the specimen substance (Figure 4B), the amount of the specimen substance that has bound to the capturing reagent is smaller, and therefore, the detection intensity of fluorescence or the like from the labeling substance on the second polymer phase of the fine particle is lower.

In the case of using a multiphase polymer fine particle including a magnetic material, the magnetic material is preferably enclosed in a polymer phase bound to the specimen substance, or a substance that can specifically bind to the specimen substance. In this case, for example, by contacting a predetermined number of multiphase polymer fine particles with the solid phase support with a magnetic field applied, the binding between the specimen substance, or the substance that can specifically bind to the specimen substance covalently binding to the multiphase polymer fine particle and the capturing reagent, or the specimen substance on the solid phase support can be achieved efficiently. Besides, detection sensitivity of a signal from the label present on the opposite surface of the fine particle in most cases can be increased.

The method according to the present invention can detect the specimen substance in the range of 0.1 pg/mL to 1000 mg/mL. The sample may be concentrated or diluted, as necessary, to be subjected to the detection method according to the present invention. In addition, the method according to the present invention can also detect two or more specimen substances in the sample at the same time. In the case of detecting two or more specimen substances at the same time, two multiphase polymer fine particles are made, and these fine particles are contacted with the solid phase support separately, successively or at the same time. In this case, it is preferable that labeling substances used for the detection be made different, and that, for example, fluorescent substances having different fluorescence wavelengths be used.

### [System of Detecting Specimen Substance]

The present invention also provides a system for detecting the specimen substance in a sample, comprising a means for capturing the specimen substance in the sample and a means for detecting the captured specimen substance.

In the system according to the present invention, the capturing means may be a solid phase support to which a reagent that specifically binds to the specimen substance is immobilized. For the solid phase support, a material normally used in the art, such as plastic, glass and silicon can be suitably used. Those having been subjected to surface treatment can be used as the solid phase support, in order to prevent nonspecific adsorption of the specimen substance. The solid phase support may be in the form of a well or a chip, for example, and in one embodiment, it may be fixed onto a channel through which the sample and the multiphase polymer fine particle are passed. For the material of the inner surface of the channel, for example, nitrocellulose membrane, silicon resin, or gold coating may be used depending on a molecule to be immobilized, although the material is not limited to the above.

Onto the solid phase support, a capturing reagent that can specifically bind to and capture the specimen substance is immobilized. The capturing reagent may be, but is not particularly limited to, for example, an antibody against the specimen substance as an antigen, a lectin that can specifically bind to a saccharide, a polynucleotide that has complementarity allowing hybridization with a nucleic acid substance. Those having ordinary skill in the art can readily recognize and appropriately select reaction conditions and the like for ensuring a specific binding between the specimen substance and the capturing reagent.

In the system according to the present invention, the detecting means is for detecting a label detectable by a binding between the capturing reagent that has not bound to the specimen substance in the sample or the specimen substance that has bound to the capturing reagent, and the multiphase polymer fine particle according to the present invention.

The system according to the present invention can be provided in the form of a small device, which is easy to use. In this case, a microchannel through which the sample and the multiphase polymer fine particle are passed may be arranged in the device, and a chip may be fixed onto the channel as a solid phase support to which a capturing reagent is immobilized. Alternatively, the configuration may be such that a microchannel in the shape of a chip (a channel chip) to which the capturing reagent is immobilized is inserted into the system having a pump and a detection system. The microchannel may have, for example, a height of the cross section in the range of about 50 µm to about 200 µm, a width in the range of about 100 µm to about 2 mm, and a length in the range of about 10 mm to 50 mm. The material of the microchannel may be a silicon resin such as polydimethylsiloxane or a plastic such as polyethylene terephthalate. The material of the chip may be plastic or glass, for example. The chip may be a disposable type. The configuration and the shape of the small device can be appropriately selected using a means normally used in the art. With no particular limitation, the small device having a size in the range of about 0.25 to 400 cm³ and a weight of 0.4 to 20 g can be suitably used.

In an aspect where detection by fluorescence is carried out, a CCD camera, a photodiode, a photomultiplier or the like that detects fluorescence from the fine particle captured on the solid phase support can be used as the detecting means. In an aspect where detection by a chemiluminescent substance is carried out, a CCD camera can be used as the detecting means. In an aspect where detection by a radioactive labeling substance is carried out, a scintillation detector or the like can be used as the detecting means. The detecting means may be configured in the same device along with the capturing means described above, or the capturing means and the detecting means may be configured separately.

Figure 5 schematically illustrates an example of a system according to the present invention. As illustrated in Figure 5, on a capturing reagent 5 immobilized onto a solid phase support 4, a multiphase polymer fine particle that has bound to a capturing reagent not binding to the specimen substance in the sample, or a multiphase polymer fine particle that has bound to a capturing reagent via the specimen substance is present (not illustrated). The amount of a signal, for example, fluorescence from a labeling substance that has bound to the second polymer phase present in a region different from that of the first polymer phase, for example, the side of the opposite hemisphere can be measured by a detecting means 6 (for example, a CCD camera). In the case of using a multiphase polymer fine particle including a magnetic material, the magnetic material is preferably enclosed in the first polymer phase. In this case, by passing the multiphase polymer fine particle through a channel with a magnetic field 7 applied to the solid phase support, the multiphase polymer fine particle can be accumulated on the channel. As a result, the binding between the specimen substance, or the substance that can specifically bind to the specimen substance covalently binding to the multiphase polymer fine particle and the capturing reagent or the specimen substance on the solid phase support can be achieved efficiently. Besides, detection sensitivity of a signal from the label present on the opposite surface of the fine particle in most cases can be increased. By using a predetermined number of multiphase polymer fine particles, the number of fine particles bound to the capturing reagent can be readily calculated from the detection result.

The system according to the present invention can detect the label in the multiphase polymer fine particle by the detecting means, thereby measuring the amount and/or concentration of the specimen substance in the sample.

Furthermore, if the specimen substance of interest has a blood concentration as an important threshold value in relation to a disease, it is also possible to have a measurement result displayed such that a warning is given when the concentration of the specimen substance in the sample is higher or lower than a predetermined threshold value.

The system according to the present invention can quickly and easily measure a variety of specimen substances in a sample with high sensitivity by combining the nano-sized multiphase polymer fine particle with the small device having a microchannel.

In the system in the form of a small device, the measurement can be achieved only by applying a sample that has a possibility of containing the specimen substance to an insertion slot of the device, and therefore, the amount of the sample required is small, and the amount of reagents used for the examination and diagnosis can also be reduced. Accordingly, reduction of costs for examination is brought about, and the system can be provided as a cheaper diagnostic agent and diagnostic equipment than examination systems conventionally used.

In addition, the system according to the present invention may be washable type for repeated use, or may be a disposable type.

### [Kit]

The present description can further provide a kit for detecting the specimen substance in a sample, including: the multiphase polymer fine particle according to the present invention, wherein the specimen substance of interest, or a substance that can specifically bind to the specimen substance has bounded in advance; a device having a capturing reagent that specifically binds to the specimen substance in the sample, and a detector for captured specimen substance; and a reagent necessary for binding reaction between the specimen substance and the capturing reagent, binding reaction of the multiphase polymer fine particle, and detection of the specimen substance. When a chemiluminescent substance is used as the detecting means, a substrate necessary for enzymatic reaction and the like may also be included in the kit.

### Examples

The present invention will be described in further detail with reference to the following Examples, but the present invention is not limited to the following Examples.

### [Preparation Example 1: Production of Magnetic Nanoparticle 1]

In 1.25 mL of chloroform, 37.5 mg of ferric oxide (Fe₂O₃) powder (particle size: 50 nm or less) was dispersed, and the resultant mixture was subjected to an ultrasonic treatment for 5 minutes. To this, 25 mg of an 8:1 random copolymer of dodecylacrylamide and dopamine methacrylamide (PS-r-PDMA with a PS:PDMA copolymerization ratio of 8:1, D81) synthesized according to the method described in Macromolecular Rapid Communications, 35(20), 1763-1769 (2014) and 1.25 mL of chloroform were added, and the resultant mixture was further subjected to an ultrasonic treatment for 5 minutes. Thereafter, the resultant mixture was allowed to react while shaking at room temperature for 12 hours. Particles were collected by bringing a neodymium magnet near to the container. Thereafter, chloroform was added for washing three times to remove unreacted D81, and the resultant product was dried under vacuum at room temperature for 3.5 hours. The product was dispersed in THF again to achieve a concentration of 1 mg/mL, and the resultant dispersion was subjected to an ultrasonic treatment for 5 minutes.

Upon carrying out a measurement by a Fourier transform infrared spectrophotometer (FT-IR) to confirm production of a complex in which Fe₂O₃ is covered with D81, as illustrated in FT-IR measurement results before the reaction with D81 (Figure 6A) and after the reaction (Figure 6B), peaks derived from alkyl and amide were observed in the iron oxide particle after the reaction. Accordingly, production of magnetic nanoparticles in the form where iron oxide particles are covered with D81 was confirmed.

The obtained magnetic nanoparticles have a particle size in the range of 100 to 300 nm, and it was confirmed that magnetic particles are covered with the polymer in an agglomerated state.

### [Preparation Example 2: Production of Magnetic Nanoparticle 2]

In a 10 mL clear vial, 90 mg of ferric oxide (Fe₂O₃) powder (particle size: 50 nm or less) was dispersed in 5 mL of DMF, and the resultant mixture was homogenized for 10 minutes with a homogenizer (intensity of 30%). To this, 30 mg of 2:1 random copolymer (D21), 4:1 random copolymer (D41) or 8:1 random copolymer (D81) of dodecylacrylamide and dopamine methacrylamide synthesized in the same manner as in Preparation Example 1 and 1 mL of THF were added, and the resultant mixture was further homogenized for 10 minutes. Thereafter, the mixture was allowed to react while shaking at the maximum shaking rate for 12 hours. The reaction mixture was transferred to a centrifuge tube made of Teflon^{®}, and subjected to centrifugation at 10,000 rpm for 15 minutes to remove supernatant. To this, 10 mL of chloroform was added, and the resultant mixture was washed three times by vortex for about 30 seconds and centrifugation in the same manner as described above. To the resultant pellet, chloroform was added to obtain a dispersion, which was transferred to a 10 mL clear vial again and vacuum dried at ordinary temperature for 2 hours. After that, the weight was measured and a THF dispersion with a particle concentration of 10 g/L or 1 g/L was made, and particles were observed with a transmission electron microscope (TEM) and the particle size distribution was measured with a Fiber-optic Dynamic Light Scattering (FDLS) photometer. Results are shown in Figures 7-1 to 7-3.

As shown in Figure 7-1, in the case of using D21, magnetic nanoparticles having a particle size in the range of about 100 to 2000 nm and an average particle size of about 500 nm were obtained. As shown in Figure 7-2, in the case of using D41, magnetic nanoparticles had a particle size in the range of about 200 to 350 nm and an average particle size of about 430 nm. In addition, as shown in Figure 7-3, in the case of using D81, magnetic nanoparticles had a particle size in the range of about 100 to about 400 nm and an average particle size of about 285 nm.

### [Example 1: Production of Magnetic Janus Particle 1]

After mixing 0.5 mL of 1 g/L solution of polystyrene having a pyrenyl group at the terminus (PS-Pyr, Mn = 3,500, Polymer Source. Inc.) in THF, 0.5 mL of 1 g/L solution of polybutadiene having an amino group at the terminus (PB-NH₂, Mn = 1,700, Polymer Source. Inc.) in THF, and 0.5 mL of 10 g/L solution of the magnetic nanoparticle in THF prepared in Preparation Example 2 in a 12 mL brown vial that has been made hydrophobic by treatment with a silane coupling agent, 1.0 mL of water was added thereto at a rate of 1 mL/min, and the resultant mixture was subjected to vortex stirring. The resultant mixture was heated at ordinary temperature for 2 hours in a vacuum oven to evaporate THF, and after cooling, a 0.2% aqueous osmium tetroxide solution was added for dyeing for 15 minutes to observe particles with a transmission and scanning electron microscope. The production process described above is schematically illustrated in Figure 8.

As a result, as illustrated in Figures 9-1 to 9-3, production of Janus particles was confirmed in which a polymer phase composed of polystyrene and a polymer phase composed of polybutadiene were separated in a visually distinguishable manner.

### [Example 2: Production of Magnetic Janus Particle 2]

Similarly as Example 1, the particle size of particles obtained using 0.1 mg/mL, 1.0 mg/mL or 5.0 mg/mL solution of polystyrene and polybutadiene in THF was confirmed. As a result, as illustrated in Figure 10, it was confirmed that, when a higher starting polymer concentration was used, the particle size became larger, if the other conditions were the same.

### [Example 3: Confirmation of Binding of Labeling Substance to Janus Particle]

The binding of a fluorescent labeling substance to one polymer phase of the magnetic Janus particle obtained in Example 1 was confirmed. As is obvious from micrographs of Figures 11A and B, a polymer region dyed with osmium tetroxide (polybutadiene) and a polymer region from which fluorescence from the pyrenyl group was detected were distinctly separated, and it was confirmed that the polymer phase composed of polystyrene was selectively labeled with fluorescence.

### [Example 4: Production of Magnetic Janus Particle 3]

After mixing 0.5 mL of 1 g/L solution of polystyrene (PS, Mn = about 10,000, Polymer Source. Inc.) in THF, 0.5 mL of 1 g/L solution of 1,2-polybutadiene having an amino group at the terminus (PB820, JSR Corporation) in THF, and 0.5 mL of 10 g/L solution of the magnetic nanoparticle in THF prepared in Preparation Example 2 in a 12 mL brown vial that has been made hydrophobic by treatment with a silane coupling agent, 1.0 mL of water was added thereto at a rate of 1 mL/min, and the resultant mixture was subjected to vortex stirring. The resultant mixture was heated at ordinary temperature for 2 hours in a vacuum oven to evaporate THF, and FITC was added to the obtained particle to allow reaction. Upon observing the obtained particle with a fluorescence microscope, fluorescence of FITC was observed in a polymer phase composed of polybutadiene on one side of the particle, indicating that FITC can be introduced site-selectively (Figure 12).

### [Example 5: Production of Non-Magnetic Janus Particle]

A non-magnetic Janus particle was produced in the same manner as in Example 4 except that the carboxy terminus polystyrene and the amino group terminus polybutadiene (both from Polymer Source. Inc.) were used and magnetic nanoparticles were not incorporated. FITC was added to the obtained particle to allow reaction. With a fluorescence microscope, fluorescence of FITC was observed in a polymer phase composed of polybutadiene, indicating that FITC can be introduced site-selectively (Figure 13).

### [Example 6: Detection of Aldosterone Using Janus Particle]

To 1 mL of an aqueous dispersion of 1 mg of a Janus particle produced in the same manner as in Example 1 except that magnetic nanoparticles were not incorporated, 1 mL of a 5 mg/mL solution of aldosterone-3-oxime in methanol obtained by selectively modifying only the 3-position of aldosterone using the method described in J. K. McKenzie and J. A. Clements, J. Clin. Endocrinol. Metab., 38(4), 622 (1974) was added, and by using about 3 mg of 1-[3-(dimethylamino)propyl]-3-carbodiimide (EDC) as a condensing agent, the resultant mixture was allowed to react overnight at room temperature under shaking. As a result, a non-magnetic Janus particle, in which aldosterone-CMO covalently bound to the polybutadiene region and the fluorescent substance was present in the polystyrene region, was obtained.

Onto the surface of each well in a 96 well plate (Nunc), an antialdosterone antibody (A2E11) was solid-phased, and a sample containing aldosterone (Toronto Research Chemicals Inc.) at a concentration of 0.1 to 100 pg/mL was added and incubated at room temperature for 5 minutes. Next, 1.0 mg/mL of the aldosterone-binding Janus particles prepared in Example 1 was added and incubated overnight at room temperature.

After washing, observation with a fluorescence microscope (Olympus Cell Dimension) at an excitation wavelength of 345 nm and a fluorescence wavelength of 450 nm was carried out, and the fluorescence (total brightness) was digitized using an imaging software. The fluorescent intensity when the aldosterone-binding Janus particle only was added was defined as 100 (B0), and the fluorescent intensity when the aldosterone-containing sample was added was defined as B to make a graph.

As a result, as illustrated in Figure 14B, it was demonstrated that when the amount of aldosterone in the sample is small, the fluorescent intensity from pyrene, the fluorescent dye binding to the Janus particles, is high, and when the amount of aldosterone in the sample becomes larger, the fluorescent intensity becomes lower accordingly.

### [Example 7: Detection of Renin Using Janus Particle]

In the presence of a condensing agent, an anti-mouse renin antibody (#AF4277, R&D Systems, Inc.) was allowed to covalently bind to a Janus particle produced in the same manner as in Example 1 except that magnetic nanoparticles are not incorporated, to prepare a Janus particle.

Meanwhile, as a capturing reagent, an anti-mouse renin antibody (#BAF4277, R&D Systems, Inc.) was immobilized onto the surface of each well in a 96 well plate (Nunc) at a concentration of 1 µg/mL in PBS buffer, and a sample containing a recombinant mouse renin (4277-AS-020, R&D Systems, Inc.) was added to each well and incubated at room temperature for 2 hours.

Next, the above-described anti-mouse renin antibody-binding Janus particles (4 x 10⁶/mL, 100 µL) was added and incubated overnight at 4°C.

After washing, observation with a fluorescence microscope at an excitation wavelength of 345 nm and a fluorescence wavelength of 450 nm was carried out to detect fluorescence. When the amount of renin in the sample is small, the amount of renin binding to the anti-mouse renin antibody on the surface of well is small and the binding of the Janus particle is also reduced, thereby leading to a low fluorescent intensity. When the amount of renin in the sample becomes larger, the obtained fluorescent intensity becomes higher. Accordingly, it was confirmed that the fluorescence depending on the concentration of renin in the sample can be detected.

### Industrial Applicability

According to the present invention, it is possible to detect a specimen substance in a sample, such as biomarkers associated with a variety of diseases, virus and pathogenic organism, and the multiphase polymer fine particle and the detection method according to the present invention can be utilized for diagnosis of diseases, monitoring of therapeutic effects and the like.

More particularly, they can be utilized for, for example, screening of patients with primary aldosteronism involved in high blood pressure, the potential number of which is estimated to be two to four million in Japan, progress judgment of nephropathy with urine examination, diagnosis of tumor markers, examination for infectious diseases from pathogenic microorganism at an airport or clinic, examination for toxins in foods, such as botulinum toxin and histamine, and examination for infectious diseases of farm animals or pet aminals.

### Reference Signs List

- 1: Amino Group
- 2: Fluorescent Substance
- 3: Magnetic Material
- 4: Solid Phase Support
- 5: Capturing Reagent
- 6: Detecting Means
- 7: Magnetic Field

All publications, patents and patent applications cited in the present specification are incorporated herein by reference in their entirety.

## Claims

1. A multiphase polymer fine particle having two or more polymer phases formed by aggregation of two or more water-insoluble polymers, respectively, at least on a surface thereof, wherein a first polymer phase can bind to a specimen substance or a substance that can specifically bind to the specimen substance, and wherein a second polymer phase has a labeling substance,
wherein:
i) the first or second polymer phase, or a third polymer phase comprises a magnetic material;
ii) the multiphase polymer fine particle has an average particle size in the range of about 100 nm to 10 µm; and
iii) the coefficient of variation in the particle size of the multiphase polymer fine particle is within 20%.

2. A multiphase polymer fine particle having a specimen substance or a substance that can specifically bind to the specimen substance, covalently binding to the first polymer phase of the fine particle according to claim 1.

3. The multiphase polymer fine particle according to claim 2, having a labeling substance covalently binding to the second polymer phase.

4. The fine particle according to any one of claims 1 to 3, wherein the specimen substance is a protein or peptide, a saccharide, a nucleic acid, a lipid or a steroidal compound.

5. A method of detecting a specimen substance in a sample, the method comprising the following steps:
a step of contacting the sample with a solid phase support onto which a capturing reagent that specifically binds to the specimen substance is immobilized;
a step of contacting the fine particle according to any one of claims 2 to 4 with the solid phase support; and
a step of detecting a label on the fine particle that has bound to the solid phase support.

6. The method according to claim 5, wherein the fine particle is a fine particle that has covalently bound to the specimen substance, and wherein the capturing reagent not bound to the specimen substance in the sample binds to the specimen substance present on the fine particle.

7. The method according to claim 5, wherein the fine particle is a fine particle covalently bound to the substance that can specifically bind to the specimen substance, and wherein the specimen substance in the sample bound to the capturing reagent binds to the substance that can specifically bind to the specimen substance present on the fine particle.

8. The method according to any one of claims 5 to 7, wherein the specimen substance is included in a biological sample.

9. The method according to any one of claims 5 to 8, wherein the capturing reagent is an antibody.

10. A system for detecting a specimen substance in a sample, comprising a means for capturing the specimen substance in the sample and a means for detecting the captured specimen substance, wherein the capturing means is a solid phase support onto which a reagent that specifically binds to the specimen substance is immobilized, and the detecting means is for detecting a label detectable by a binding between the capturing reagent not bound to the specimen substance in the sample or the specimen substance bound to the capturing reagent, and the fine particle according to any one of claims 2 to 4.

11. The system according to claim 10, wherein the system is provided in the form of a small device.

12. The system according to claim 11, wherein the solid phase support is a chip fixed onto a microchannel in a device through which the sample is passed.

13. The system according to claim 11 or 12, wherein the detecting means is a CCD camera that detects fluorescence from a fine particle captured on the solid phase support.

14. The system according to any one of claims 10 to 13, wherein the system measures the amount and/or concentration of the specimen substance.

## Patentansprüche

1. Mehrphasiges Polymer-Feinteilchen, das zwei oder mehr Polymerphasen umfasst, die durch Aggregation von jeweils zwei oder mehr wasserunlöslichen Polymeren auf zumindest einer Oberfläche davon ausgebildet sind, wobei eine erste Polymerphase in der Lage ist, sich an eine Probensubstanz oder an eine Substanz zu binden, die in der Lage ist, sich spezifisch an die Probensubstanz zu binden, und wobei eine zweite Polymerphase eine Markierungssubstanz aufweist, wobei:
i) die erste oder die zweite Polymerphase oder eine dritte Polymerphase ein magnetisches Material umfasst;
ii) das mehrphasige Polymer-Feinteilchen eine mittlere Teilchengröße im Bereich von etwa 100 nm bis 10 µm aufweist; und
iii) der Variationskoeffizient in der Teilchengröße des mehrphasige Polymer-Fein-teilchens innerhalb von 20 % liegt.

2. Mehrphasiges Polymer-Feinteilchen, das eine Probensubstanz oder eine Substanz, die in der Lage ist, sich spezifisch an die Probensubstanz zu binden, umfasst, die sich kovalent an die erste Polymerphase eines Feinteilchens nach Anspruch 1 bindet.

3. Mehrphasiges Polymer-Feinteilchen nach Anspruch 2, das eine Markierungssubstanz umfasst, die sich kovalent an die zweite Polymerphase bindet.

4. Feinteilchen nach einem der Ansprüche 1 bis 3, wobei die Probensubstanz ein Protein oder Peptid, ein Saccharid, eine Nucleinsäure, ein Lipid oder eine Steroidverbindung ist.

5. Verfahren zum Nachweisen einer Probensubstanz in einer Probe, wobei das Verfahren die folgenden Schritte umfasst:
einen Schritt des Inkontaktbringens der Probe mit einem Festphasenträger, auf dem ein Einfangreagens immobilisiert ist, das sich spezifisch an die Probensubstanz bindet;
einen Schritt des Inkontaktbringens eines Feinteilchens nach einem der Ansprüche 2 bis 4 mit dem Festphasenträger; und
einen Schritt des Nachweisens einer Markierung auf dem Feinteilchen, das sich an den Festphasenträger gebunden hat.

6. Verfahren nach Anspruch 5, wobei das Feinteilchen ein Feinteilchen ist, das sich kovalent an die Probensubstanz gebunden hat, und wobei sich das Einfangreagens, das nicht an die Probensubstanz in der Probe gebunden ist, an die auf dem Feinteilchen vorhandene Probensubstanz bindet.

7. Verfahren nach Anspruch 5, wobei das Feinteilchen ein Feinteilchen ist, das kovalent an die Substanz gebunden ist, die in der Lage ist, sich spezifisch an die Probensubstanz zu binden, und wobei sich die Probensubstanz in der Probe, die an das Einfangreagens gebunden ist, an die Substanz bindet, die in der Lage ist, sich spezifisch an die auf dem Feinteilchen vorhandene Probensubstanz zu binden.

8. Verfahren nach einem der Ansprüche 5 bis 7, wobei die Probensubstanz in einer biologischen Probe umfasst ist.

9. Verfahren nach einem der Ansprüche 5 bis 8, wobei das Einfangreagens ein Antikörper ist.

10. System zum Nachweisen einer Probensubstanz in einer Probe, das ein Mittel zum Einfangen der Probensubstanz in der Probe und ein Mittel zum Nachweisen der eingefangenen Probensubstanz umfasst, wobei das Einfangmittel ein Festphasenträger ist, auf dem ein Reagens immobilisiert ist, das sich spezifisch an die Probensubstanz bindet, und das Nachweismittel zum Nachweisen einer Markierung dient, die durch eine Bindung zwischen dem Einfangreagens, das nicht an die Probensubstanz in der Probe gebunden ist, oder der an das Einfangreagens gebundenen Probensubstanz und einem Feinteilchen nach einem der Ansprüche 2 bis 4 nachweisbar ist.

11. System nach Anspruch 10, wobei das System in Form einer kleinen Vorrichtung bereitgestellt ist.

12. System nach Anspruch 11, wobei der Festphasenträger ein auf einem Mikrokanal fixierter Chip in einer Vorrichtung ist, durch den die Probe geleitet wird.

13. System nach Anspruch 11 oder 12, wobei das Nachweismittel eine CCD-Kamera ist, die Fluoreszenz von einem Feinteilchen nachweist, das auf dem Festphasenträger eingefangen ist.

14. System nach einem der Ansprüche 10 bis 13, wobei das System die Menge und/oder Konzentration der Probensubstanz misst.

## Revendications

1. Particule fine de polymère à plusieurs phases présentant deux phases de polymère ou plus formées par accumulation de deux polymères insolubles dans l'eau ou plus, respectivement, au moins sur une surface de celle-ci, dans laquelle une première phase polymère peut se lier à une substance spécimen ou une substance qui peut se lier spécifiquement à la substance spécimen, et dans laquelle une deuxième phase de polymère présente une substance de marquage,
dans laquelle :
i) la première ou la deuxième phase de polymère, ou une troisième phase de polymère comprend un matériau magnétique ;
ii) la particule fine de polymère à plusieurs phases présente une taille de particule moyenne dans la plage d'environ 100 nm à 10 µm ; et
iii) le coefficient de variation de la taille de particule de la particule fine de polymère à plusieurs phases est inférieur à 20 %.

2. Particule fine de polymère à plusieurs phases présentant une substance spécimen ou une substance qui peut se lier spécifiquement à la substance spécimen, se liant de manière covalente à la première phase de polymère de la particule fine selon la revendication 1.

3. Particule fine de polymère à plusieurs phases selon la revendication 2, présentant une substance de marquage se liant de manière covalente à la deuxième phase de polymère.

4. Particule fine selon l'une quelconque des revendications 1 à 3, dans laquelle la substance spécimen est une protéine ou un peptide, un saccharide, un acide nucléique, un lipide ou un composé stéroïdien.

5. Procédé de détection d'une substance spécimen dans un échantillon, le procédé comprenant les étapes suivantes :
une étape de mise en contact de l'échantillon avec un support en phase solide sur lequel un réactif de capture qui se lie spécifiquement à la substance spécimen est immobilisé ;
une étape de mise en contact de la particule fine selon l'une quelconque des revendications 2 à 4 avec le support en phase solide ; et
une étape de détection d'un marqueur sur la particule fine qui s'est liée au support en phase solide.

6. Procédé selon la revendication 5, dans lequel la particule fine est une particule fine qui s'est liée de manière covalente à la substance spécimen, et dans lequel le réactif de capture non lié à la substance spécimen dans l'échantillon se lie à la substance spécimen présente sur la particule fine.

7. Procédé selon la revendication 5, dans lequel la particule fine est une particule fine liée de manière covalente à la substance qui peut se lier spécifiquement à la substance spécimen, et dans lequel la substance spécimen dans l'échantillon liée au réactif de capture se lie à la substance qui peut se lier spécifiquement à la substance spécimen présente sur la particule fine.

8. Procédé selon l'une quelconque des revendications 5 à 7, dans lequel la substance spécimen est incluse dans un échantillon biologique.

9. Procédé selon l'une quelconque des revendications 5 à 8, dans lequel le réactif de capture est un anticorps.

10. Système pour détecter une substance spécimen dans un échantillon, comprenant un moyen pour capturer la substance spécimen dans l'échantillon et un moyen pour détecter la substance spécimen capturée, dans lequel le moyen de capture est un support en phase solide sur lequel un réactif qui se lie spécifiquement à la substance spécimen est immobilisé, et le moyen de détection est destiné à détecter un marqueur détectable par une liaison entre le réactif de capture non lié à la substance spécimen dans l'échantillon ou la substance spécimen liée au réactif de capture, et la particule fine selon l'une quelconque des revendications 2 à 4.

11. Système selon la revendication 10, dans lequel le système est fourni sous la forme d'un petit dispositif.

12. Système selon la revendication 11, dans lequel le support en phase solide est une puce fixée sur un microcanal dans un dispositif à travers lequel l'échantillon est amené à passer.

13. Système selon la revendication 11 ou 12, dans lequel le moyen de détection est une caméra CCD qui détecte une fluorescence à partir d'une particule fine capturée sur le support en phase solide.

14. Système selon l'une quelconque des revendications 10 à 13, dans lequel le système mesure la quantité et/ou la concentration de la substance spécimen.
